Europäisches Patentamt

European Patent Office

Office européen des brevets

(11) Veröffentlichungsnummer: **0 252 531 B1**

(12) **EUROPÄISCHE PATENTSCHRIFT**

(45) Veröffentlichungstag der Patentschrift: **02.12.92**

(21) Anmeldenummer: **87111726.3**

(22) Anmeldetag: **05.06.87**

(60) Veröffentlichungsnummer der früheren
Anmeldung nach Art. 76 EPÜ:

(51) Int. Cl.⁵: **C07K 15/00**, //C12N15/38

(54) Strukturelles Phosphoprotein (pp 150) des menschlichen Cytomegalovirus, seine Herstellung und Verwendung.

(30) Priorität: **12.06.86 DE 3619718**

(43) Veröffentlichungstag der Anmeldung:
**13.01.88 Patentblatt 88/02**

(45) Bekanntmachung des Hinweises auf die
Patenterteilung:
**02.12.92 Patentblatt 92/49**

(84) Benannte Vertragsstaaten:
**AT BE CH DE ES FR GB GR IT LI LU NL SE**

(56) Entgegenhaltungen:

**JOURNAL OF VIROLOGY, Band 61, Nr. 5, Mai
1987, Seiten 1358-1367, American Society for
Microbiology; G. JAHN et al.: "Map position
and nucleotide sequence of the gene for the
large structural phosphoprotein of human
cytomegalovirus"**

**VIROLOGY, Band 128, 1983, Seiten 391-406,
Academic Press, Inc.; W. GIBSON: "Protein
counterparts of human and simian cytomegaloviruses"**

(73) Patentinhaber: **BEHRINGWERKE Aktiengesellschaft
Postfach 1140
W-3550 Marburg 1(DE)**

(72) Erfinder: **Jahn, Gerhard, Dr.
Effeltrichersrasse 11
W-8524 Neunkirchen(DE)**
Erfinder: **Scholl,Birgit-Christine
Schlehenweg 4
W-8521 Uttenreuth(DE)**
Erfinder: **Bröker, Michael, Dr.
Lindenweg 16
W-3550 Marburg(DE)**
Erfinder: **Mach, Michael, Dr.
Geschwister-Scholl-Strasse 10
W-8520 Erlangen(DE)**
Erfinder: **Fleckenstein, Bernard, Prof.
Schlaifhausen 93
W-8551 Hausen(DE)**
Erfinder: **Traupe, Bernd
Marquardstrasse 3D
W-8551 Hausen(DE)**

VIROLOGY, Band 132, 1984, Seiten 325-338, Academic Press, Inc.; B. NOWAK et al.: "Characterization of monoclonal antibodies and polyclonal immune sera directed against human cytomegalovirus virion proteins"

PROCEEDINGS OF THE NATIONAL ACADEMY OF SCIENCE OF THE USA, Band 82, Februar 1985, Seiten 1266-1270; E.S. MOCARSKI et al.: "Precise localization of genes on large animal virus genomes: use of lambdagt 11 and monoclonal antibodies to map the gene for a cytomegalovirus protein family"

PROCEEDINGS OF THE NATIONAL ACADEMY OF SCIENCE OF THE USA, Band 80, März 1983, Seiten 1194-1198; R.A. YOUNG et al.: "Efficient isolation of genes by using antibody probes"

(74) Vertreter: Meyer-Dulheuer, Karl-Hermann, Dr. et al
HOECHST Aktiengesellschaft Postfach 3540
W-6200 Wiesbaden(DE)

## Beschreibung

Ein phosphoryliertes Strukturprotein von etwa 150 kd (pp 150) ist ein Bestandteil von gereinigten Virion-Partikeln. Nach W. Gibson, Virology 128 (1983) 391 - 406, ist es ein Konstituent der Matrix. Wegen seiner stark immunogenen Eigenschaften eignet es sich als Diagnostikum.

Die Erfindung betrifft pp 150 und immunogene Teile dieses Proteins, deren gentechnische Herstellung und ihre Verwendung als immunologisches Reagens, beispielsweise im ELISA. Bevorzugte Ausgestaltungen der Erfindung in ihren verschiedenen Aspekten werden im folgenden näher erläutert bzw. in den Patentansprüchen definiert.

Es wurde gefunden, daß man mit einem monospezifischen Kaninchen-Antiserum gegen pp 150 die gewünschten Klone aus einer HCMV-cDNA-Genbank identifizieren kann. Hierzu wurde eine Probe des gesamten viralen Proteins einer präparativen SDS-Polyacrylamid-Gelelektrophorese unterworfen und die einzelnen Proteinbanden mit Hilfe des Farbstoffs $^R$Coomassie (ICI) Brillant-Blau sichtbar gemacht. Das Protein mit dem Molekulargewicht 150 kd wurde herausgeschnitten, extrahiert und zur Immunisierung von Kaninchen verwendet. Das gewonnene Antiserum zeigte im "Western Blot"-Test Reaktion mit dem 150 kd Protein. Dieses Serum wurde für das "Screening" der cDNA-Genbank eingesetzt.

Zur Herstellung der Genbank wurde aus mit HCMV, Stamm Ad 169, infizierten menschlichen Vorhaut-Fibroblastenzellen 96 bis 120 Stunden nach der Infektion die poly(A)$^+$-RNA isoliert, in ds-DNA überführt und ohne Größenfraktionierung in den handelsüblichen Phagen-Expressionsvektor λgt11 eingefügt. Hierzu wurde der Vektor mit Eco-RI gespalten und mit alkalischer Phosphatase (aus Kälberdarm) behandelt, um die intramolekulare Religation zu unterdrücken. Durch Anfügen von EcoRI-Linkern wurde die cDNA zwischen die Phagenarme eingefügt und in vitro verpackt. Aus 100 ng ds-cDNA wurde so eine Genbank erhalten, die etwa $5 \bullet 10^5$ unabhängige Rekombinanten und 18 % Wildtyp-Phagen enthielt.

Das "Screening" der Genbank erfolgte nach der Methode von R. A. Young und R. W. Davis, Proc. Natl. Acad. Sci. USA 80 (1983) 1194 - 1198, jedoch mit der Abwandlung, daß Meerrettich-Peroxidase an Protein A gekoppelt wurde und 4-Chlor-1-naphthol als Detektionssystem diente, unter Einsatz der vorstehend beschriebenen monospezifischen Kaninchen-Antikörper. Bei diesem "Immunoscreening" werden die auf Nitrozellulosefiltern vorhandenen Kolonien vorsichtig lysiert, mit oben beschriebenen monospezifischen Kaninchen-Antikörpern inkubiert und nach Entfernen ungebundener Reaktanden positive Plaques mit dem genannten modifizierten Detektionssystem nachgewiesen.

Von 150 000 untersuchten Plaques wurden 8 positive Signale erhalten. Ein Klon mit einer Insertion von etwa 300 Basen wurde für die weitere Charakterisierung ausgewählt; er erhielt die Bezeichnung BB 8.

Der E. coli-Stamm Y 1089 wurde mit dem rekombinanten Phagen infiziert und die Synthese des β-Galactosidase-Proteins durch Zugabe von Isopropylthiogalactosid (IPTG) induziert. Hierbei wurde ein Fusions-Protein gebildet, das deutlich größer als die Galactosidase (118 kd) ist. Es wird weder in nichtinfizierten Zellen noch in infizierten, aber nichtinduzierten Zellen gefunden. Sowohl menschliche HCMV-positive Seren als auch das Kaninchen-Anti-pp 150-Serum reagierten nur mit diesem Protein in BB 8-infizierten, induzierten Zellen, erkannten jedoch weder Proteine in nichtinfizierten oder nichtinduzierten infizierten Zellen. Es ergibt sich somit, daß der rekombinante Klon BB 8 ein Fusionsprotein mit einem HCMV-Protein-Anteil synthetisiert.

Mit dem Fusionsprotein aus BB 8 wurde ein Kaninchen immunisiert und mit dem Antiserum wurden "Western Blot"-Analysen mit HCMV-Proteinen durchgeführt. Es reagierte nur das pp 150.

Die cDNA-Insertion von 300 bp wurde nun verwendet, um das Gen für pp 150 im Virus-Genom zu lokalisieren: Hierfür wurde die cDNA-Insertion von 300 bp mit 8 Cosmid-Klonen hybridisiert, die das gesamte Genom von HCMV umspannen (B. Fleckenstein et al., Gene 18 (1982) 39 - 46). Die Cosmide pCM 1015 und pCM 1017, die überlappend die HindIII-J-, -N und Y-Fragmente enthalten, hybridisierten mit der cDNA. Eine eingehendere "Southern Blot"-Analyse dieser Region begrenzte das HCMV-DNA-Fragment auf ein 1,5 kb EcoRI-PstI-Fragment, das im EcoRI-Y-Fragment lokalisiert ist, und zwar benachbart zum C-Fragment.

"Northern Blot"-Analysen mit "später" RNA und $^{32}$P-markierter DNA des Klones BB 8 (in M13 umkloniert) ergaben ein abundantes Transkript von 6,2 kb.

"Northern Blot"-Analysen mit unterschiedlich klonierten viralen DNA-Fragmenten aus dem HindIII-J- und -N-Fragment ergaben verschiedene Größenklassen von "später" RNA. Das stärkste Signal ergab sich mit einer RNA-Größenklasse von 6,2 kb.

Von allen untersuchten Strukturproteinen wurde pp 150 in "Western blot"-Analysen am zuverlässigsten von menschlichen HCMV-positiven Seren erkannt. Dabei reagieren sowohl IgM-positive als auch IgG-positive Seren von unterschiedlichsten Patienten, beispielsweise kongenital infizierten Kindern, AIDS-erkrankten u.a. symptomatischen und asymptomatischen Personen.

Da pp 150 in den Mengen, die für Diagnostika erforderlich sind, nur unter großem technischem Aufwand zu isolieren wäre, ist die erfindungsgemäße gentechnische Herstellungsweise besonders vorteilhaft. Es zeigte sich, daß nicht nur von eukaryotischen Zellen exprimierte Produkte antigen wirken, sondern auch Expressionsprodukte von Bakterien. Da Bakterien keine Phosphoproteine erzeugen, war nicht zu erwarten, daß auch bakteriell hergestelltes HCMV-pp 150 oder Teile davon stark immunogen wirken. Es zeigte sich jedoch, daß auch solche Proteine von entsprechenden Seren ebenso eindeutig erkannt werden wie authentisches pp 150.

Erfindungsgemäß kann somit in prokaryotischen oder eukaryotischen Zellen, beispielsweise Hefezellen, menschlichen oder animalen Zellen, hergestelltes pp 150 oder immunogene Teile davon als Reagens für einen HCMV-Antikörpernachweis verwendet werden, beispielsweise im ELISA.

Beispiel

Das XhoII-PstI-Fragment, das innerhalb des HCMV-EcoRI-Y Fragmentes liegt und somit für Teile des pp 150 codiert, wurde in den Expressionsvektor pBD 2 (M. Bröker, Gene Anal. Techn. 3 (1986) 53 - 57) ligiert, nachdem der Vektor mit BamHI und Pst gespalten worden war.

Nach Transformation des erhaltenen Hybridplasmids in E. coli BMH 71-18 wurden Klone isoliert, deren Plasmid-DNA das zu erwartende Restriktionsmuster besaßen. Nach Induktion des lac-Promotors mit Isopropyl-ß-D-thiogalacto-pyranosid (IPTG) exprimierten die Klone große Mengen eines Fusionsproteins mit einem pp 150-Anteil.

In "Western blot"-Analysen wurde das rekombinante Fusionsprotein, nicht aber das durch pBD 2 codierte Kontrollprotein in allen getesteten HCMV-positiven humanen Seren ebenso eindeutig erkannt wie authentisches pp 150.

Das neue Plasmid, das für dieses ß-Galactosidase-pp 150 Fusionsprotein codiert, wird im folgenden pXP1 genannt. Aus E. coli-Zellen, die den Vektor pXP1 enthalten, wurde nach Induktion mit IPTG, das durch pXP1 codierte Fusionsprotein isoliert und zur Immunisierung von Kaninchen verwendet. Serum, das nach dreimaliger Immunisierung gewonnen worden war, reagierte in Western blot-Analysen mit Proteinbanden von ca. 150.000 d von HCMV infizierten Zellextrakten, nicht aber mit Kontrollextrakten. Somit sind Antikörper, die gegen das bakteriell synthetisierte pp 150 hergestellt worden sind, in der Lage, authentisches pp 150 zu erkennen. Ferner konnte das anti-pXP1-Serum dazu benutzt werden, bereits zwei bis drei Tage nach Infektion von Zellkulturen, HCMV mittels Immunfluoreszenz nachzuweisen, während der cytopathische Effekt erst nach zehn bis vierzehn Tagen erkennbar wird. Somit kann ein Serum, das gegen rekombinant hergestelltes pp 150 gewonnen wurde, als diagnostisches Mittel eingesetzt werden.

**Patentansprüche**
**Patentansprüche für folgende Vertragsstaaten : BE, CH, DE, FR, GB, IT, LI, LU, NL, SE**

1. Immunogenes Protein, dadurch gekennzeichnet, daß es durch Expression des Gens, das für ein strukturelles Phosphoprotein mit einem Molgewicht von etwa 150 kd (pp 150) aus humanem Cytomegalovirus (HCMV) kodiert und das im Bereich der Fragmente HindIII-Y/N des Genomes von HCMV, Stamm Ad 169, liegt, in prokaryotischen oder eukaryotischen Zellen hergestellt wird, wobei das genannte immunogene Protein nicht identisch ist mit CMV-Isolaten der menschlichen Stämme AD169, Davis und Towne, mit den Wildtyp Isolaten C-1, 751 und Charcot-Marie-Toothe und mit den CMV-Isolaten aus Rhesus- und Vervetaffen und dem Colburn Affen-CMV-Isolat.

2. Immunogenes Protein nach Anspruch 1, dadurch gekennzeichnet, daß es durch Expression des genannten Gens, das auf einem 1,5 kb EcoRI-PstI-Fragment innerhalb des EcoRI-Y-Fragments aus dem Genom von HCMV, Stamm Ad 169, liegt, hergestellt wird.

3. Immunogene Teile des strukturellen Phosphoproteins mit einem Molgewicht von etwa 150 kd (pp 150) aus humanem Cytomegalovirus (HCMV), erhältlich durch Expression von Teilen des Gens, das im Bereich der Fragmente HindIII-Y/N des Genomes von HCMV, Stamm Ad 169, liegt.

4. Immunogene Teile des pp 150, erhältlich durch Expression von Teilen des Gens, das auf einem 1,5 kb EcoRI-PstI-Fragment innerhalb des EcoRI-Y-Fragments aus dem Genom von HCMV, Stamm Ad 169, liegt.

5. Verfahren zur Herstellung von pp 150 oder immunogenen Teilen davon, dadurch gekennzeichnet, daß man das Gen ganz oder teilweise zur Expression bringt, das in dem offenen Leserahmen der HindIII-Y/N-Fragmente aus dem Genom von HCMV, Stamm Ad 169, liegt.

6. Verfahren nach Anspruch 5, dadurch gekennzeichnet, daß man ein 1,5 kb EcoRI-PstI-Fragment aus dem EcoRI-Y-Fragment einsetzt.

**7.** Verwendung der Proteine nach Anspruch 1 bis 4 bzw. der nach Anspruch 5 bis 6 erhaltenen Proteine oder immunogenen Teile dieser Proteine als in vitro diagnostisches Mittel.

**8.** Verwendung der Proteine nach Anspruch 1 bis 4 bzw. der nach Anspruch 5 bis 6 erhaltenen Proteine oder immunogenen Teile dieser Proteine im ELISA-Text.

**9.** Verwendung der Proteine nach Anspruch 1 bis 4 bzw. der nach Anspruch 5 bis 6 erhaltenen Proteine oder immunogenen Teile dieser Proteine zur Herstellung eines Impfstoffes.

**Patentansprüche für folgende Vertragsstaaten : AT, ES, GR**

**1.** Verfahren zur Herstellung eines strukturellen Phosphoproteins mit einem Molgewicht von etwa 150 kd (pp150) aus humanem Cytomegalovirus (HCMV) oder immunogenen Teilen davon, dadurch gekennzeichnet, daß man das Gen ganz oder teilweise zur Expression bringt, das in dem offenen Leserahmen der HindIII-Y/N-Fragmente aus dem Genom von HCMV, Stamm Ad 169, liegt.

**2.** Verfahren nach Anspruch 1, dadurch gekennzeichnet, daß man ein 1,5 kb EcoRI-PstI-Fragment aus dem EcoRI-Y-Fragment einsetzt.

**Claims**
**Claims for the following Contracting States : BE, CH, DE, FR, GB, IT, LI, LU, NL, SE**

**1.** An immunogenic protein which is prepared by expression of the gene which codes for a structural phosphoprotein with a molecular weight of about 150 kd (pp 150) from human cytomegalovirus (HCMV) and which is located in the region of the fragments HindIII Y/N of the genome of HCMV, strain Ad 169, in prokaryotic or eukaryotic cells, said immunogenic protein not being identical to CMV isolates of human strains Ad 169, Davis and Towne, to the wild-type isolates C-1, 751 and Charcot-Marie-Toothe and to the CMV isolates from rhesus and vervet monkeys and to the Colburn monkey CMV isolate.

**2.** An immunogenic protein as claimed in claim 1, which is prepared by expression of said gene which is located on a 1.5 kb EcoRI-PstI fragment inside the EcoRI Y fragment from the genome of HCMV, strain Ad 169.

**3.** Immunogenic parts of the structural phosphoprotein with a molecular weight of about 150 kd (pp 150) from human cytomegalovirus (HCMV), obtainable by expression of parts of the gene which is located in the region of the fragments HindIII Y/N of the genome of HCMV, strain Ad 169.

**4.** Immunogenic parts of pp 150, obtainable by expression of parts of the gene which is located on a 1.5 kb EcoRI-PstI fragment inside the EcoRI Y fragment from the genome of HCMV, strain Ad 169.

**5.** A process for the preparation of pp 150 or immunogenic parts thereof, which comprises bringing about expression of the gene, in whole or in part, which is located in the open reading frame of the HindIII Y/N fragments from the genome of HCMV, strain Ad 169.

**6.** The process as claimed in claim 5, wherein use is made of a 1.5 kb EcoRI-PstI fragment from the EcoRI Y fragment.

**7.** The use of the proteins as claimed in claim 1 to 4 or of the proteins obtained as claimed in claim 5 or 6, or immunogenic parts of these proteins, as in vitro diagnostic agent.

**8.** The use of the proteins as claimed in claim 1 to 4 or of the proteins obtained as claimed in claim 5 or 6, or immunogenic parts of these proteins, in an ELISA.

**9.** The use of the proteins as claimed in claim 1 to 4 or of the proteins obtained as claimed in claim 5 or 6, or immunogenic parts of these proteins, for preparing a vaccine.

**Claims for the following Contracting States : AT, ES, GR**

**1.** A process for the preparation of a structural phosphoprotein with a molecular weight of about 150 kd (pp 150) from human cytomegalovirus (HCMV) or immunogenic parts thereof, which comprises bringing about expression of the gene, in whole or in part, which is located in the open reading frame of the HindIII Y/N fragments from the genome of HCMV, strain Ad 169.

**2.** The process as claimed in claim 4, wherein use is made of a 1.5 kb EcoRI-PstI fragment from the EcoRI Y fragment.

**Revendications**
**Revendications pour les Etats contractants**

suivants : **BE, CH, DE, FR, GB, IT, LI, LU, NL, SE**

1. Protéine immunogène, caractérisée en ce qu'elle est produite dans des cellules procaryotes ou eucaryotes par expression du gène qui code pour une phosphoprotéine structurale ayant une masse moléculaire d'environ 150 kd (pp 150), provenant du cytomégalovirus humain (HCMV), et qui se trouve dans la région des fragments HindIII-Y/N du génome de HCMV, souche Ad 169, ladite protéine immunogène n'étant pas identique à des isolats de CMV des souches humaines AD169, Davis et Towne, aux isolats de type sauvage C-1, 751 et Charcot-Marie-Toothe et aux isolats de CMV provenant de singes rhésus et Vervet et à l'isolat de CMV de singe Colburn.

2. Protéine immunogène selon la revendication 1, caractérisée en ce qu'elle est produite par expression dudit gène qui se trouve sur un fragment EcoRI-PstI de 1,5 kb à l'intérieur du fragment EcoRI-Y provenant du génome de HCMV, souche Ad 169.

3. Parties immunogènes de la phosphoprotéine structurale ayant une masse moléculaire d'environ 150 kd (pp 150) provenant du cytomégalovirus humain (HCMV), pouvant être obtenues par expression de parties du gène qui se trouve dans la région du fragment HindIII-Y/N du génome de HCMV, souche Ad 169.

4. Parties immunogènes de la pp 150, pouvant être obtenues par expression de parties du gène qui se trouve sur un fragment EcoRI-PstI de 1,5 kb à l'intérieur du fragment EcoRI-Y provenant du génome de HCMV, souche Ad 169.

5. Procédé pour la production de pp 150 ou de parties immunogènes de celles-ci, caractérisé en ce que l'on amène à l'expression en partie ou en totalité le gène qui se trouve dans le cadre de lecture ouvert des fragments HindIII-Y/N provenant du génome de HCMV, souche Ad 169.

6. Procédé selon la revendication 5, caractérisé en ce que l'on utilise un fragment EcoRI-PstI de 1,5 kb provenant du fragment EcoRI-Y.

7. Utilisation des protéines selon l'une des revendications 1 à 4 ou des protéines obtenues selon la revendication 5 ou 6, ou de parties immunogènes de ces protéines, en tant qu'agent de diagnostic.

8. Utilisation des protéines selon l'une des revendications 1 à 4 ou des protéines obtenues selon la revendication 5 ou 6, ou de parties immunogènes de ces protéines, dans l'essai ELISA.

9. Utilisation des protéines selon l'une des revendications 1 à 4 ou des protéines obtenues selon la revendication 5 ou 6, ou de parties immunogènes de ces protéines, pour la fabrication d'un vaccin.

**Revendications pour les Etats contractants suivants: AT, ES, GR**

1. Procédé pour la production d'une phosphoprotéine structurale ayant une masse moléculaire d'environ 150 kd (pp 150), provenant du cytomégalovirus humain (HCTMV), ou de parties immunogènes de celle-ci, caractérisé en ce que l'on amène à l'expression en partie ou en totalité le gène qui se trouve dans le cadre de lecture ouvert des fragments HindIII-Y/N provenant du génome de HCMV, souche Ad 169.

2. Procédé selon la revendication 5, caractérisé en ce que l'on utilise un fragment EcoRI-PstI de 1,5 kb provenant du fragment EcoRI-Y.